# FASCICULE DE BREVET EUROPEEN

(11) **EP 1 637 121 B1**
(45) Date de publication et mention de la délivrance du brevet: **31.12.2008**
(21) Numéro de dépôt: 05291660.8
(22) Date de dépôt: 03.08.2005
(51) Int. Cl.: A61K 8/34, A61K 8/41, A61Q 5/12, A61K 8/895

(54) **Composition cosmétique à base d'un tensioactif cationique, d'une silicone aminée, d'un alcool gras et d'un diol**
Kosmetische Zusammensetzung auf der Basis eines kationischen Tensids, eines Aminosilikons, eines Fettalkohols und eines Diols
Cosmetic composition based on a cationic surfactant, an aminosilicone, a fatty alcohol, and a diol

(30) Priorité: 08.09.2004 FR 0451990
(43) Date de publication de la demande: 22.03.2006
(73) Titulaire: L'ORÉAL, 75008 Paris (FR)
(72) Inventeur: Lazzeri, Pascale, 92300 Levallois Perret (FR); Decoster, Sandrine, 95210 Saint Gratien (FR)
(74) Mandataire: Le Blainvaux Bellegarde, Françoise

(56) Documents cités:
- EP-A- 1 468 665
- EP-A- 1 491 180
- US-A- 4 910 013
- US-A1- 2003 003 073
- US-A1- 2004 166 084

## Description

La présente invention est relative à de nouvelles compositions cosmétiques, notamment capillaires, comprenant au moins un tensioactif cationique, au moins une silicone aminée, au moins un alcool gras, et au moins un diol particulier. Elle vise également l'utilisation de ces compositions en cosmétique, et en particulier l'utilisation de ces compositions comme après-shampooing.

Les après-shampooings classiques comprenant un tensioactif cationique et des alcools gras permettent d'obtenir de bonnes propriétés cosmétiques des cheveux. Toutefois, l'utilisateur a parfois la sensation que ses cheveux sont lourds, peu légers, et qu'ils regraissent facilement, tout particulièrement si ses cheveux sont fins, de plus pour certains types de cheveux les propriétés cosmétiques ne sont pas suffisantes. En outre, la répartition du produit est parfois difficile.

US-A-4 910 013 (KANAMARU ET AL) décrit (voir exemple 4) une composition cosmétique comprenant:
- un tensioactif cationique quaternaire (1 ou 2)
- une silicone aminée (8)
- alcool gras (cetyl alcohol)
- un diol (5).

La Demanderesse vient de découvrir de façon surprenante que des compositions comprenant au moins un tensioactif cationique particulier, au moins une silicone aminée, quaternisée ou non, au moins un alcool gras, et au moins un diol ayant 6 atomes de carbone dans des rapports particuliers entre les différents constituants, permettaient de remédier aux inconvénients mentionnés ci-dessus.

Ella a en particulier constaté que ces compositions permettaient un assouplissement de la fibre c'est-à-dire rendait la fibre moins rêche et plus malléable.

Elle a également constaté que lors de l'application, les compositions se répartissaient et s'étalaient facilement des racines jusqu'aux pointes et que le rinçage était particulièrement aisé.

Elle a enfin remarqué que ces compositions conféraient de bonnes propriétés cosmétiques aux cheveux, et notamment le démêlage, le lissage, que les cheveux étaient légers et regraissaient moins vite, et que les cheveux se mettaient en forme très facilement, et durablement.

La présente invention a donc pour objet une composition cosmétique non colorante, notamment capillaire, caractérisée en ce qu'elle comprend, dans un milieu cosmétiquement acceptable, au moins un tensioactif cationique choisi parmi les sels d'ammonium quaternaires, au moins une silicone aminée, quaternisée ou non, au moins un alcool gras, et au moins un diol ayant 6 atomes de carbone, le rapport des concentrations en poids alcool gras/tensioactif cationique étant supérieur ou égal à 1, le rapport des concentrations en poids tensioactif cationique/silicone étant supérieur ou égal à 0,85 et le rapport des concentrations en poids alcool gras/silicone allant de 2 à 5 le diol étant présent à une teneur de 0,25 à 5%, l'alcool gras étant choisi parmi l'alcool cétylique, l'alcool stéarylique, l'alcool oléïque, la composition contenant moins de 0,5% de colorant d'oxydation.

Un autre objet de l'invention concerne un procédé cosmétique capillaire mettant en oeuvre les compositions selon l'invention.

L'invention a également pour objet l'utilisation des compositions selon l'invention pour le traitement cosmétique des cheveux et du cuir chevelu.

Un autre objet de l'invention est l'utilisation de la composition pour le conditionnement des matières kératiniques telles que les cheveux.

D'autres caractéristiques, aspects, objets et avantages de l'invention apparaîtront encore plus clairement à la lecture de la description et des exemples qui suivent.

Par « au moins un », on comprendra « un ou plusieurs », C'est-à-dire un, deux, trois ou plus.

Par "milieu cosmétiquement acceptable", on entend un milieu compatible avec toutes les matières kératiniques telles que la peau, les cheveux, les ongles, les cils, les sourcils, les lèvres et toute autre zone du corps et du visage.

Par « composition non colorante », on entend une composition ne contenant essentiellement pas de colorant d'oxydation, c'est-à-dire moins de 0,5% en poids par rapport au poids total de la composition, de préférence moins de 0,1% en poids et plus particulièrement ne contenant pas de colorant d'oxydation.

Le milieu cosmétiquement acceptable comprend de préférence de l'eau ou un ou plusieurs solvants cosmétiquement acceptable, de préférence hydrophyle ou des mélanges eau-solvant(s). Ces solvants sont de préférence des alcools et notamment les monoalcools, linéaires ou ramifiés en C1-C6, comme l'éthanol, le tertiobutanol, le n-butanol, l'isopropanol ou le n-propanol.

L'eau ou le mélange d'eau et de solvants cosmétiquement acceptable peut être présent dans la composition selon l'invention en une teneur allant de 30% à 99% en poids, par rapport au poids total de la composition, et de préférence de 60% à 90% en poids.

Parmi les sels d'ammonium quaternaire on peut citer les sels d'alkylpyridinium, les sels d'ammonium d'imidazoline, les sels de diammonium quaternaire, les sels d'ammonium contenant au moins une fonction ester.

A titre de sels d'ammonium quaternaire, on peut notamment citer, par exemple :
- ceux qui présentent la formule générale (I) suivante : 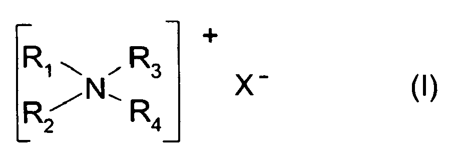
dans laquelle les symboles R1 à R4, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, notamment en C12-C22, alcoxy, polyoxyalkylène (C2-C6), alkylamide, alkyl(C12-C22)amidoalkyle(C2-C6), alkyl(C12-C22)acétate, hydroxyalkyle, comportant environ de 1 à 30 atomes de carbone ; X- est un anion choisi dans le groupe des halogénures, phosphates, acétates, lactates, alkyl(C2-C6)sulfates, alkyl- ou alkylaryl-sulfonates ;
- les sels d'ammonium quaternaire de l'imidazoline comme, par exemple, ceux de formule (II) suivante : 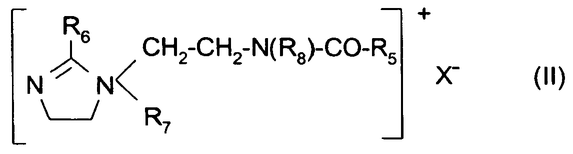 dans laquelle R5 représente un radical alcényle ou alkyle comportant de 8 à 30 atomes de carbone par exemple dérivés des acides gras du suif ou du coprah, R6 représente un atome d'hydrogène, un radical alkyle en C1-C4 ou un radical alcényle ou alkyle comportant de 8 à 30 atomes de carbone, R7 représente un radical alkyle en C1-C4 , R8 représente un atome d'hydrogène, un radical alkyle en C1-C4, X est un anion choisi dans le groupe des halogénures, phosphates, acétates, lactates, alkylsulfates, alkyl-ou-alkylarylsulfonates. De préférence, R5 et R6 désignent un mélange de radicaux alcényle ou alkyle comportant de 12 à 21 atomes de carbone par exemple dérivés des acides gras du suif, R7 désigne méthyle, R8 désigne hydrogène. Un tel produit est par exemple le Quaternium-27(CTFA 1997) ou le Quaternium-83 (CTFA 1997) commercialisés sous les dénominations "REWOQUAT®" W 75, W90, W75PG, W75HPG par la société WITCO,
- les sels de diammonium quaternaire de formule (III) : 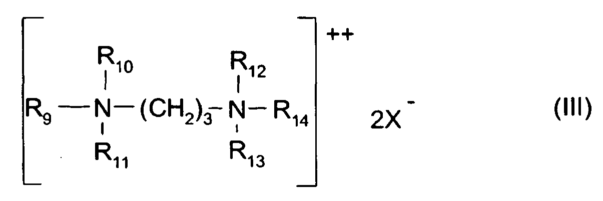 dans laquelle R9 désigne un radical aliphatique comportant environ de 16 à 30 atomes de carbone, R10, R11, R12, R13 et R14, identiques ou différents sont choisis parmi l'hydrogène ou un radical alkyle comportant de 1 à 4 atomes de carbone, et X- est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IV) suivante : 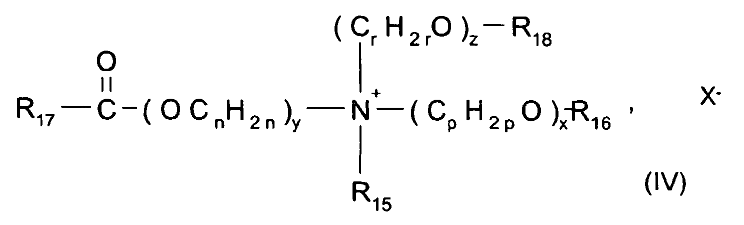 dans laquelle :
   R15 est choisi parmi les radicaux alkyles en C1-C6 et les radicaux hydroxyalkyles ou dihydroxyalkyles en C1-C6 ;
   R16 est choisi parmi :
- le radical 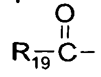
   - les radicaux R20 hydrocarbonés en C1-C22, linéaires ou ramifiés, saturés ou insaturés,
   - l'atome d'hydrogène,
   R18 est choisi parmi :

- le radical 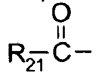
- les radicaux R22 hydrocarbonés en C1-C6, linéaires ou ramifiés, saturés ou insaturés,
- l'atome d'hydrogène,
R17, R19 et R21, identiques ou différents, sont choisis parmi les radicaux hydrocarbonés en C7-C21, linéaires ou ramifiés, saturés ou insaturés ;
r, n et p, identiques ou différents, sont des entiers valant de 2 à 6 ;
y est un entier valant de 1 à 10 ;
x et z, identiques ou différents, sont des entiers valant de 0 à 10;
X- est un anion simple ou complexe, organique ou inorganique ;
sous réserve que la somme x + y + z vaut de 1 à 15, que lorsque x vaut 0 alors R16 désigne R20 et que lorsque z vaut 0 alors R18 désigne R22.

Les radicaux alkyles R15 peuvent être linéaires ou ramifiés, et plus particulièrement linéaires.

De préférence, R15 désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R16 est un radical R20 hydrocarboné, il peut être long et avoir de 12 à 22 atomes de carbone, ou court et avoir de 1 à 3 atomes de carbone.

Lorsque R18 est un radical R22 hydrocarboné, il a de préférence 1 à 3 atomes de carbone.

Avantageusement, R17, R19 et R21, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.

De préférence, x et z, identiques ou différents, valent 0 ou 1.

Avantageusement, y est égal à 1.

De préférence, r, n et p, identiques ou différents, valent 2 ou 3, et encore plus particulièrement sont égaux à 2.

L'anion X- est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement dans la composition selon l'invention, les sels d'ammonium de formule (IV) dans laquelle :
- R15 désigne un radical méthyle ou éthyle,
- x et y sont égaux à 1 ;
- z est égal à 0 ou 1 ;
- r, n et pt sont égaux à 2 ;
   R16 est choisi parmi :

- le radical 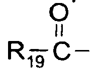
   - les radicaux méthyle, éthyle ou hydrocarbonés en C14-C22,
   - l'atome d'hydrogène ;
   - R18 est choisi parmi :

- le radical 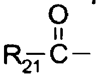
   - l'atome d'hydrogène ;
   - R17, R19 et R21, identiques ou différents, sont choisis parmi les radicaux hydrocarbonés en C13-C17, linéaires ou ramifiés, saturés ou insaturés, et de préférence parmi les radicaux alkyles et alcényles en C13-C17, linéaires ou ramifiés, saturés ou insaturés.

Avantageusement, les radicaux hydrocarbonés sont linéaires.

On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire mentionnés ci-dessus, on préfère utiliser ceux répondant à la formule (I). On peut notamment citer d'une part, les sels (et notamment les méthosulfates) de dipalmitoyléthylhydroxyéthylméthylammonium, les sels (et notamment les chlorures) de tétraalkylammonium comme, par exemple, les sels (et notamment les chlorures) de dialkyldiméthylammonium ou d'alkyltriméthyl-ammonium dans lesquels le radical alkyle comporte environ de 12 à 22 atomes de carbone, en particulier les sels (et notamment les chlorures) de béhényltriméthylammonium, de distéaryldiméthylammonium, de cétyltriméthylammonium, de benzyldiméthylstéarylammonium ou encore, d'autre part, les sels (et notamment les chlorures) de palmitylamidopropyltriméthylammonium ou les sels (et notamment les chlorures) de stéaramidopropyldiméthyl-(myristylacétate)-ammonium, et notamment le chlorure de stéaramidopropyldiméthyl-(myristyl acétate)-ammonium commercialisé sous la dénomination CERAPHYL® 70 par la société VAN DYK.

Les tensioactifs cationiques encore plus particulièrement préférés dans la composition de l'invention sont choisis parmi les sels d'ammonium quaternaire, et en particulier parmi le chlorure de béhényltriméthylammonium, le méthosulfate de dipalmitoyléthyl hydroxyéthyl méthyl ammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

Le ou les tensioactifs cationiques peuvent être présents dans la composition à une teneur comprise entre 0,01 et 10%, de préférence entre 0,1 et 5%, encore de préférence entre 0,2 et 4% en poids du poids total de la composition.

Selon l'invention, on désigne par silicone aminée toute silicone comportant au moins une amine primaire, secondaire, tertiaire ou un groupement ammonium quaternaire. On peut ainsi citer :
a) les polysiloxanes dénommés dans le dictionnaire CTFA "amodiméthicone" et répondant à la formule : 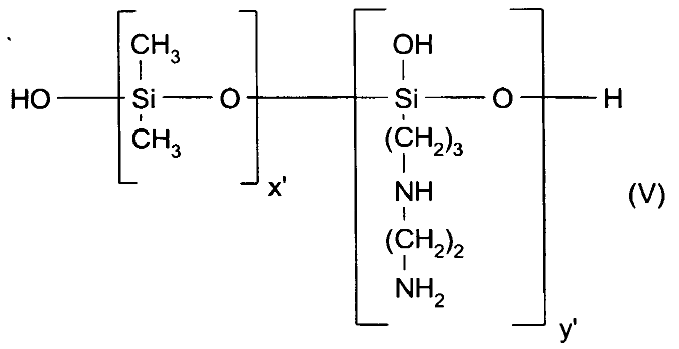 dans laquelle x' et y' sont des nombres entiers dépendant du poids moléculaire, généralement tels que ledit poids moléculaire moyen en poids est compris entre 5 000 et 500 000 environ ;
b) les silicones aminées répondant à la formule :

   R'aG3-a-Si(OSiG2)n-(OSiGbR'2-b)m-O-SiG3-a-R'a (VI)

   dans laquelle :
   G est un atome d'hydrogène, ou un groupement phényle, OH, ou alkyle en C1-C8, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -CqH2qL dans laquelle q est un nombre de 2 à 8 et L est un groupement aminé éventuellement quaternisé choisi parmi les groupements :
      -NR"-Q-N'(R")2
      -N(R")2
      -N⊕(R")3 A-
      -NH⊕(R")2 A-
      -NH2⊕(R") A-
      -N(R")-Q-N⊕R"H2 A-
      -NR"-Q-N⊕(R")2H A-
      -NR"-Q-N⊕(R")3 A-,
      dans lesquels R" peut désigner hydrogène, phényle, benzyle, ou un radical hydrocarboné saturé monovalent, par exemple un radical alkyle ayant de 1 à 20 atomes de carbone ; Q désigne un groupement de formule CᵣH₂ᵣ, linéaire ou ramifié, r étant un entier allant de 2 à 6, de préférence de 2 à 4 ; et A- représente un ion halogénure tel que par exemple fluorure, chlorure, bromure ou iodure.
   Un produit correspondant à cette définition est la silicone dénommée "triméthylsilylamodiméthicone", répondant à la formule : 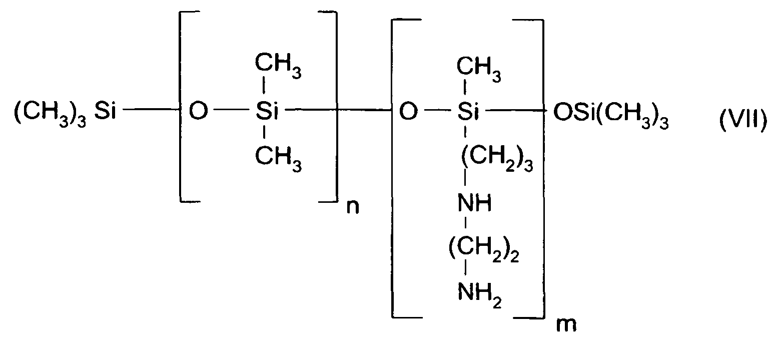 dans laquelle n et m ont les significations données ci-dessus (cf formule VI).
   De tels polymères sont décrits par exemple dans la demande de brevet EP-A-95238.
c) les silicones aminées répondant à la formule : 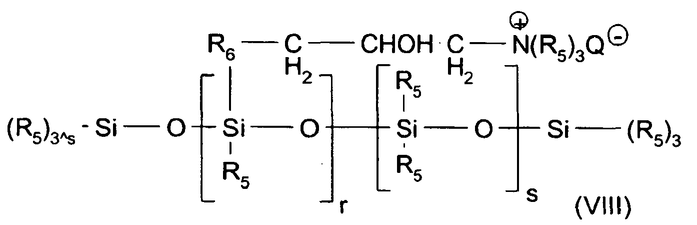 dans laquelle :
   R5 représente un radical hydrocarboné monovalent ayant de 1 à 18 atomes de carbone, et en particulier un radical alkyle en C1-C18, ou alcényle en C2-C18, par exemple méthyle ;
   R6 représente un radical hydrocarboné divalent, notamment un radical alkylène en C1-C18 ou un radical alkylèneoxy divalent en C1-C18, par exemple en C1-C8 relié au Si par une liaison SiC;
   Q- est un anion tel qu'un ion halogénure, notamment chlorure ou un sel d'acide organique (acétate ...);
   r représente une valeur statistique moyenne de 2 à 20 et en particulier de 2 à 8 ;
   s représente une valeur statistique moyenne de 20 à 200 et en particulier de 20 à 50.
   De telles silicones aminées sont décrites plus particulièrement dans le brevet US 4 185 087.
d) les silicones ammonium quaternaire de formule : 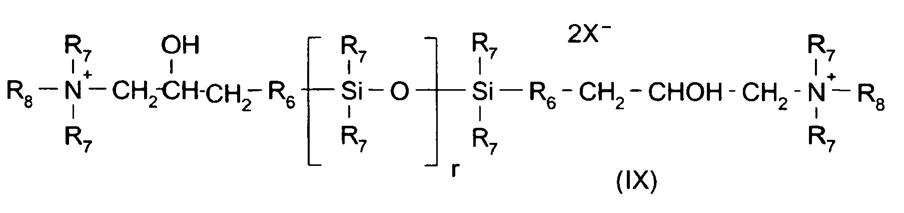 dans laquelle :
   R7, identiques ou différents, représentent un radical hydrocarboné monovalent ayant de 1 à 18 atomes de carbone, et en particulier un radical alkyle en C1-C18, un radical alcényle en C2-C18 ou un cycle comprenant 5 ou 6 atomes de carbone, par exemple méthyle ;
   R6 représente un radical hydrocarboné divalent, notamment un radical alkylène en C1-C18 ou un radical alkylèneoxy divalent en C1-C18, par exemple en C1-C8 relié au Si par une liaison SiC;
   R8, identiques ou différents, représentent un atome d'hydrogène, un radical hydrocarboné monovalent ayant de 1 à 18 atomes de carbone, et en particulier un radical alkyle en C1-C18, un radical alcényle en C2-C18 , un radical -R6-NHCOR7 ;
   X- est un anion tel qu'un ion halogénure, notamment chlorure ou un sel d'acide organique (acétate ...);
   r représente une valeur statistique moyenne de 2 à 200 et en particulier de 5 à 100 ;
   Ces silicones sont par exemple décrites dans la demande EP-A-0530974.
e) les silicones aminées de formule (X) : 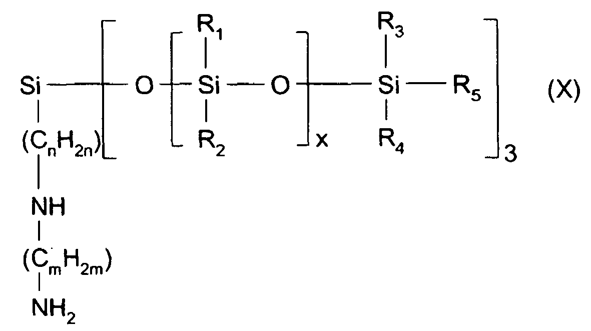 dans laquelle :
   - R1, R2, R3 et R4, identiques ou différents, désignent un radical alkyle en C1-C4 ou un groupement phényle,
   - R5 désigne un radical alkyle en C1-C4 ou un groupement hydroxyle,
   - n est un entier variant de 1 à 5,
   - m est un entier variant de 1 à 5,
   et dans laquelle x est choisi de manière telle que l'indice d'amine soit compris entre 0,01 et 1 meq/g.

Les silicones particulièrement préférées conformément à l'invention sont les polysiloxanes à groupements aminés tels que les amodiméthicones ou les triméthylsilylamodiméthicones (CTFA 4ème édition 1997), et encore plus particulièrement les silicones à groupements ammonium quaternaire.

La ou les silicones aminée peuvent être présentes dans la composition à une teneur comprise entre 0,01 et 6%, de préférence entre 0,1 et 3%, encore de préférence entre 0,4 et 2% en poids du poids total de la composition.

L'alcool gras peut être

l'alcool cétylique, l'alcool stéarylique, et l'alcool oléique et leurs mélanges.

L'alcool gras peut représenter un mélange d'alcools gras, ce qui signifie que dans un produit commercial peuvent coexister plusieurs espèces d'alcools gras, sous forme d'un mélange.

Avantageusement, l'alcool gras est solide ou pâteux à la température de 20°C. Par « alcool gras solide ou pâteux à 20°C », on entend au sens de la présente invention un alcool gras présentant une viscosité mesurée avec un rhéomètre avec un taux de cisaillement de 1s⁻¹ supérieure ou égale à 1 Pa.s.

Le ou les alcools gras peuvent être présents dans la composition à une teneur allant de 0,1 à 15%, de préférence de 0,5 à 10%, encore de préférence de 2 à 8% en poids du poids total de la composition.

Par diol ayant 6 atomes de carbone au sens de l'invention, on entend tout composé hydrocarboné comprenant 2 fonctions hydroxy et 6 atomes de carbone.

Le diol peut être linéaire ou ramifié et comprendre des fonctions éthers.

Le diol est de préférence choisi parmi l'hexane diol-1,6, le dipropylèneglycol et leurs mélanges.

Le ou les diols peuvent être présents dans la composition à une teneur allant de 0,25 à 5%, encore de préférence de 0,5 à 3% en poids par rapport au poids total de la composition.

Le rapport des concentrations en poids alcool gras/tensioactif cationique va de préférence de 1 à 10 et plus particulièrement de 1 à 5.

Le rapport des concentrations en poids tensioactif cationique/silicone va de 0,85 à 5.

Le rapport des concentrations en poids alcool gras/silicone va de 2 à 5.

La composition selon l'invention peut contenir en outre au moins un additif choisi parmi les parfums, les filtres UV, les tensioactifs non ioniques, anioniques, amphotères ou zwitterioniques, les conservateurs, les protéines, les vitamines, les polymères non ioniques, anioniques, cationiques, amphotères ou zwitterioniques, les agents antipelliculaires, les agents anti-chute des cheveux, les colorants, les pigments, les réducteurs, les cires végétales, les céramides, les provitamines, les polymères non ioniques, anioniques, cationiques, amphotères ou zwitterioniques, les huiles minérales, végétales ou synthétiques, les silicones non aminées, les cires végétales, les céramides, les polyols autres que ceux de l'invention et tout autre additif classiquement utilisé dans les compositions cosmétiques.

Ces additifs sont présents dans la composition à des teneurs avantageusement comprises entre 0,001 et 20% en poids du poids total de la composition. La quantité précise de chaque additif est fonction de sa nature et est déterminée facilement par l'homme du métier, et dépendra de l'application capillaire choisie.

L'homme du métier veillera à choisir les éventuels additifs et leur quantité de manière à ce qu'ils ne nuisent pas aux propriétés des compositions de la présente invention.

Les compositions selon l'invention peuvent se présenter sous forme de liquides fluides ou épaissis, de gels, de crèmes, ou d'émulsions simples ou multiples. Les compositions de traitement cosmétique des matières kératiniques conformes à l'invention peuvent aussi se présenter sous forme d'une mousse et peuvent être utilisées en application rincée ou non. Dans ce cas, elles peuvent être conditionnées dans un dispositif aérosol.

Les compositions peuvent être utilisées, par exemple, dans des shampoings, des après-shampooings, dans des produits de décoloration ou de permanente, produits de coiffage, soins rincés, masques de soin profond, gels douches, lotions ou crèmes de traitement du cuir chevelu, ou encore déposées sur des lingettes.

L'invention a également pour objet l'utilisation des compositions selon l'invention pour le traitement cosmétique des cheveux et du cuir chevelu.

Un autre objet de l'invention est l'utilisation de la composition pour le conditionnement des matières kératiniques telles que les cheveux et plus particulièrement comme après-shampoing.

La présente invention a également pour objet un procédé de traitement cosmétique dans lequel la composition cosmétique selon l'invention est appliquée sur les cheveux, humides ou secs, cette application étant éventuellement suivie d'un rinçage. L'application de la composition s'effectue avantageusement après un shampooing.

Dans une forme de réalisation préférée de l'invention, les compositions selon l'invention sont utilisées comme après-shampooings pour le traitement des cheveux et du cuir chevelu.

Elles sont appliquées, dans ce cas-là, sur des cheveux humides ou secs, dans des quantités efficaces pour les traiter, cette application étant éventuellement suivie d'un rinçage.

Les exemples suivants sont destinés à illustrer l'invention. Les quantités sont exprimées en matière active sauf indication contraire.

### Exemple 1

On a préparé une composition A d'après-shampooing selon invention :

| | Composition A | Composition B |
|---|---|---|
| Alcool cétylique | 3 g | 3 g |
| Chlorure de béhényl triméthyl ammonium | 1,43 g MA | 1,43 g MA |
| Dipropylèneglycol | 0,57 gMA | - |
| Propylèneglycol | - | 0,57 gMA |
| Mélange de Myristate/Palmitate/Stéarate de Myristyle/Cétyle/Stéaryle | 1 g | 1 g |
| Polydiméthylsiloxane à groupement aminoéthyliminopropyle en émulsion aqueuse à 35% (DC939 de DOW CORNING) | 0,94 g MA | 0,94 g MA |
| Lanoline (LANOLINE USP 10/40 | 0,15g | 0,15g |
| Parfum, Conservateurs | qs | qs |
| Agent de pH qs | pH 4 ± 0,5 | pH 4 ± 0,5 |
| Eau | Qsp 100g | Qsp 100g |

Les compositions A et B sont appliquées sur les cheveux, cette application étant suivie d'un rinçage.

La composition A se répartit plus facilement sur les cheveux de la racine à la pointe.

Le rinçage de la composition A se fait plus facilement. Les cheveux sont plus assouplis sous l'eau.

On observe que les cheveux soumis à l'application de la composition A selon l'invention sont plus faciles à démêler et plus lisses que les cheveux soumis à l'application de la composition B.

### Exemple 2

On a préparé une composition A d'après shampooing selon invention :

| | Composition A | Composition B |
|---|---|---|
| Alcool cétylstéarylique | 5 g | 5 g |
| Chlorure de béhényl triméthyl ammonium | 3,2g MA | 3,2g MA |
| Dipropylèneglycol | 1,27 gMA | - |
| Isopropanol | - | 1,27 gMA |
| Mélange de Myristate/Palmitate/Stéarate de Myristyle/Cétyle/Stéaryle | 1g | 1g |
| Polydiméthylsiloxane à groupement aminoéthyliminopropyle en émulsion aqueuse à 35% (DC939 de DOW CORNING) | 1,05 g MA | 1,05 g MA |
| Parfum, Conservateurs | qs | qs |
| Agent de pH qs | pH 4 ± 0,5 | pH 4 ± 0,5 |
| Eau | Qsp 100g | Qsp 100g |

Les compositions A et B sont appliquées sur les cheveux, cette application étant suivie d'un rinçage.

La composition A se répartit plus facilement sur les cheveux de la racine à la pointe.

Le rinçage de la composition A se fait plus facilement. Les cheveux sont plus assouplis sous l'eau.

On observe que les cheveux soumis à l'application de la composition A selon l'invention sont plus faciles à démêler et plus lisses que les cheveux soumis à l'application de la composition B.

### Exemple 3

On a préparé une composition A d'après shampooing selon invention :

| | Composition A | Composition B |
|---|---|---|
| Alcool cétylstéarylique | 7 g | 7 g |
| Chlorure de béhényl triméthyl ammonium | 5 g MA | 5 g MA |
| Dipropylèneglycol | 2 gMA | - |
| Isopropanol | - | 2 gMA |
| Mélange de Myristate/Palmitate/Stéarate de Myristyle/Cétyle/Stéaryle | 1,5 g | 1,5 g |
| Polydiméthylsiloxane à groupement aminoéthyliminopropyle en émulsion aqueuse à 35% (DC939 de DOW CORNING) | 1,75 g MA | 1,75 g MA |
| Glycérol | 3 g | 3 g |
| Parfum, Conservateurs | qs | qs |
| Agent de pH qs | pH 4,5 ± 0,5 | pH 4,5 ± 0,5 |
| Eau | Qsp 100g | Qsp 100g |

Les compositions A et B sont appliquées sur les cheveux, cette application étant suivie d'un rinçage.

Le rinçage de la composition A se fait plus facilement.

On observe que les cheveux soumis à l'application de la composition A selon l'invention sont plus faciles à démêler et plus lisses que les cheveux soumis à l'application de la composition B.

### Exemple 4

On a préparé une composition A d'après shampooing selon invention et une composition comparative B:

| | Composition A | Composition B |
|---|---|---|
| Alcool cétylstéarylique | 7 g | 7 g |
| Chlorure de béhényl triméthyl ammonium | 5 g MA | 5 g MA |
| Hexanediol-1,6 | 1,67 gMA | - |
| Isopropanol | - | 1,67 gMA |
| Mélange de Myristate/Palmitate/Stéarate de Myristyle/Cétyle/Stéaryle | 1,5 g | 1,5 g |
| Polydiméthylsiloxane à groupement aminoéthyliminobutyle en émulsion aqueuse à 58% (DC2-8299 de DOW CORNING) | 1,75 g MA | 1,75 g MA |
| Glycérol | 3 g | 3 g |
| Parfum, Conservateurs | qs | qs |
| Agent de pH qs | pH 4,5 ± 0,5 | pH 4,5 ± 0,5 |
| Eau | Qsp 100g | Qsp 100g |

Les compositions A et B sont appliquées sur les cheveux, cette application étant suivie d'un rinçage.

Le rinçage de la composition A se fait plus facilement. Les cheveux sont plus assouplis sous l'eau.

On observe que les cheveux soumis à l'application de la composition A selon l'invention sont plus faciles à démêler et plus lisses que les cheveux soumis à l'application de la composition B.

## Revendications

1. Composition cosmétique non colorante, notamment capillaire, **caractérisée en ce qu'**elle comprend, dans un milieu cosmétiquement acceptable, au moins un tensioactif cationique choisi parmi les sels d'ammonium quaternaire, au moins une silicone aminée, au moins un alcool gras et au moins un diol ayant 6 atomes de carbone, le rapport des concentrations en poids alcool gras/tensioactif cationique étant supérieur ou égal à 1, le rapport des concentrations en poids tensioactif cationique/silicone étant supérieur ou égal à 0,85 et le rapport des concentrations en poids alcool gras/silicone allant de 2 à 5,
le diol étant présent dans la composition à une teneur allant de 0,25 à 5% en poids du poids total de la composition,
l'alcool gras étant choisi parmi l'alcool cétylique, l'alcool stéarylique, l'alcool oléique et leurs mélanges.
la composition contenant moins de 0,5% en poids de colorant d'oxydation par rapport au poids total de la composition.

2. Composition selon la revendication 1, **caractérisée en ce que** les sels d'ammonium quaternaire sont choisis parmi :
- ceux qui présentent la formule générale (I) suivante : 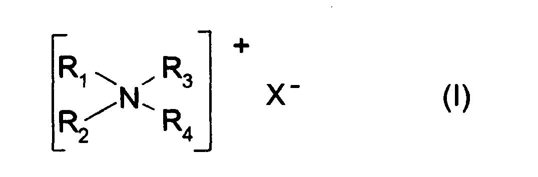 dans laquelle les symboles R1 à R4, qui peuvent être identiques ou différents, représentent un radical aliphatique, linéaire ou ramifié, comportant de 1 à 30 atomes de carbone, ou un radical aromatique tel que aryle ou alkylaryle ; X- est un anion choisi dans le groupe des halogénures, phosphates, acétates, lactates, alkyl(C2-C6)sulfates, alkyl- ou alkylarylsulfonates ;
- les sels d'ammonium quaternaire de l'imidazoline de formule (II) suivante : 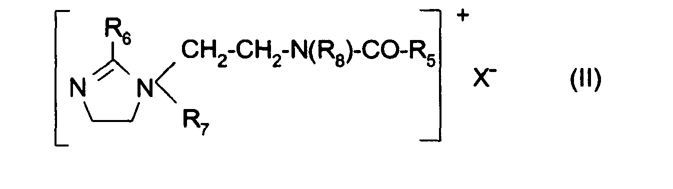 dans laquelle R5 représente un radical alcényle ou alkyle comportant de 8 à 30 atomes de carbone, R6 représente un atome d'hydrogène, un radical alkyle en C1-C4 ou un radical alcényle ou alkyle comportant de 8 à 30 atomes de carbone, R7 représente un radical alkyle en C1-C4 , R8 représente un atome d'hydrogène, un radical alkyle en C1-C4, X est un anion choisi dans le groupe des halogénures, phosphates, acétates, lactates, alkylsulfates, alkyl-ou-alkylarylsulfonates,
- les sels de diammonium quaternaire de formule (III) : 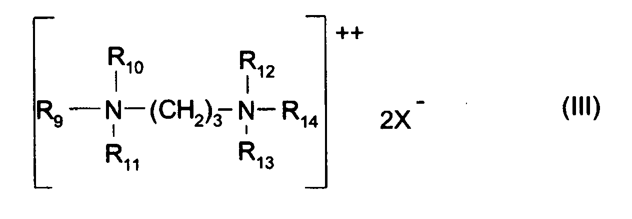 dans laquelle R9 désigne un radical aliphatique comportant de 16 à 30 atomes de carbone, R10, R11, R12, R13 et R14, identiques ou différents sont choisis parmi l'hydrogène ou un radical alkyle comportant de 1 à 4 atomes de carbone, et X- est un anion choisi dans le groupe des halogénures, acétates, phosphates, nitrates, éthylsulfates et méthylsulfates ;
- les sels d'ammonium quaternaire contenant au moins une fonction ester, de formule (IV) suivante : 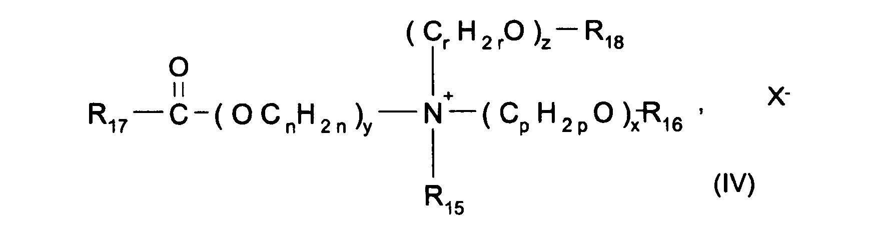 dans laquelle :
R15 est choisi parmi les radicaux alkyles en C1-C6 et les radicaux hydroxyalkyles ou dihydroxyalkyles en C1-C6 ;
R16 est choisi parmi :
- le radical 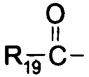
- les radicaux R20 hydrocarbonés en C1-C22, linéaires ou ramifiés, saturés ou insaturés,
- l'atome d'hydrogène,
R18 est choisi parmi :
- le radical 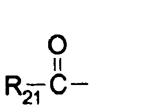
- les radicaux R22 hydrocarbonés en C1-C6, linéaires ou ramifiés, saturés ou insaturés,
- l'atome d'hydrogène,
R17, R19 et R21, identiques ou différents, sont choisis parmi les radicaux hydrocarbonés en C7-C21, linéaires ou ramifiés, saturés ou insaturés ;
r, n et p, identiques ou différents, sont des entiers valant de 2 à 6 ;
y est un entier valant de 1 à 10 ;
x et z, identiques ou différents, sont des entiers valant de 0 à 10 ;
X- est un anion simple ou complexe, organique ou inorganique ;
sous réserve que la somme x + y + z vaut de 1 à 15, que lorsque x vaut 0 alors R16 désigne R20 et que lorsque z vaut 0 alors R18 désigne R22.

3. Composition selon la revendication 2, **caractérisée en ce que** le composé de formule (IV) est choisi parmi les sels de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges.

4. Composition selon la revendication 2, **caractérisée en ce que** le tensioactif de formule (I) est choisi parmi les sels de dipalmitoyléthylhydroxyéthylméthylammonium, les sels de béhényltriméthylammonium, de distéaryldiméthylammonium, de cétyl triméthylammonium, de benzyldiméthylstéarylammonium, les sels de palmitylamidopropyltriméthylammonium, les sels de stéaramido propyl diméthyl-(myristylacétate)-ammonium.

5. Composition selon l'une quelconque des revendications précédentes, **caractérisée en ce que** le tensioactif cationique est choisi parmi le chlorure de béhényltriméthylammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

6. Composition selon l'une quelconque des revendications précédentes, **caractérisée en ce que** le tensioactif cationique est présent dans la composition à une teneur comprise entre 0,01 et 10%, de préférence entre 0,1 et 5%, et encore de préférence entre 0,2 et 4% en poids du poids total de la composition.

7. Composition selon l'une quelconque des revendications précédentes, **caractérisée en ce que** la silicone aminée est choisie parmi :
a) les polysiloxanes dénommés dans le dictionnaire CTFA "amodiméthicone" et répondant à la formule : 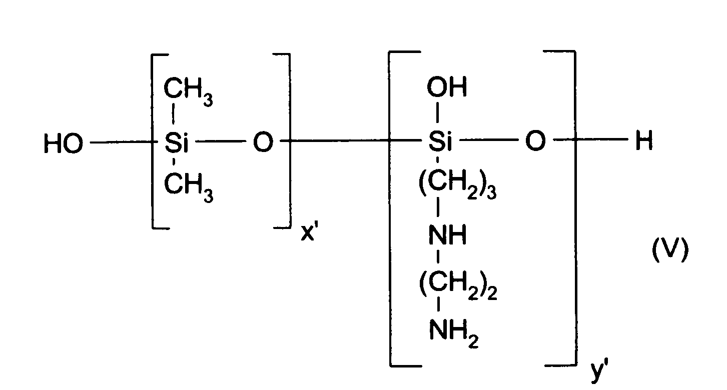 dans laquelle x' et y' sont des nombres entiers tels que le poids moléculaire moyen en poids est compris entre 5 000 et 500 000 ;
b) les silicones aminées répondant à la formule :
R'aG3-a-Si(OSiG2)n-(OSiGbR'2-b)m-O-SiG3-a-R'a (VI)
dans laquelle :
G est un atome d'hydrogène, ou un groupement phényle, OH, ou alkyle en C1-C8,
a désigne le nombre 0 ou un nombre entier de 1 à 3,
b désigne 0 ou 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R' est un radical monovalent de formule -CqH2qL dans laquelle q est un nombre de 2 à 8 et L est un groupement aminé éventuellement quaternisé choisi parmi les groupements :
-NR"-Q-N'(R")2
-N(R")2
-N⊕(R")3 A-
-NH⊕(R")2 A-
-NH2⊕(R") A-
-N(R")-Q-N⊕R"H2 A-
-NR"-Q-N⊕(R")2H A-
-NR"-Q-N⊕(R")3 A-
dans lesquels R" peut désigner hydrogène, phényle, benzyle, ou un radical hydrocarboné saturé monovalent; Q désigne un groupement de formule CᵣH₂ᵣ, linéaire ou ramifié, r étant un entier allant de 2 à 6, de préférence de 2 à 4 ;et A- représente un ion halogénure,
c) les silicones aminées répondant à la formule : 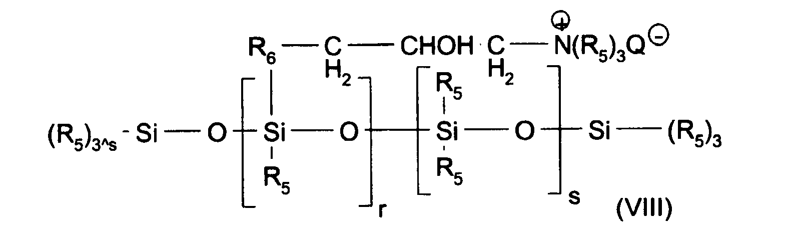 dans laquelle :
R5 représente un radical hydrocarboné monovalent ayant de 1 à 18 atomes de carbone, et en particulier un radical alkyle en C1-C18, ou alcényle en C2-C18 ;
R6 représente un radical hydrocarboné divalent, notamment un radical alkylène en C1-C18 ou un radical alkylèneoxy divalent en C1-C18 ;
Q- est un anion tel qu'un ion halogénure ou un sel d'acide organique;
r représente une valeur statistique moyenne de 2 à 20 et en particulier de 2 à 8 ;
s représente une valeur statistique moyenne de 20 à 200 et en particulier de 20 à 50.
d) les silicones ammonium quaternaire de formule : 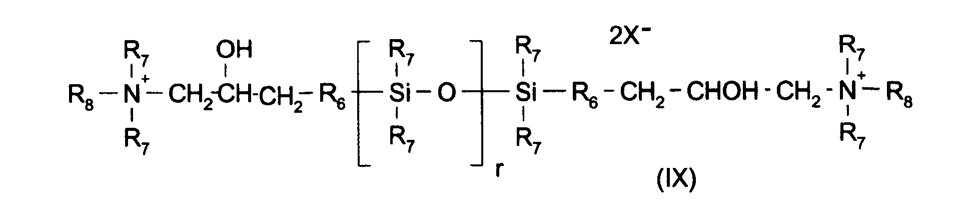 dans laquelle :
R7, identiques ou différents, représentent un radical hydrocarboné monovalent ayant de 1 à 18 atomes de carbone, et en particulier un radical alkyle en C1-C18, un radical alcényle en C2-C18 ou un cycle comprenant 5 ou 6 atomes de carbone ;
R6 représente un radical hydrocarboné divalent, notamment un radical alkylène en C1-C18 ou un radical alkylèneoxy divalent en C1-C18;
R8, identiques ou différents, représentent un atome d'hydrogène, un radical hydrocarboné monovalent ayant de 1 à 18 atomes de carbone, et en particulier un radical alkyle en C1-C18, un radical alcényle en C2-C18 , un radical -R6-NHCOR7 ;
X- est un anion tel qu'un ion halogénure ou un sel d'acide organique;
r représente une valeur statistique moyenne de 2 à 200 et en particulier de 5 à 100 ;
e) les silicones aminées de formule (X) : 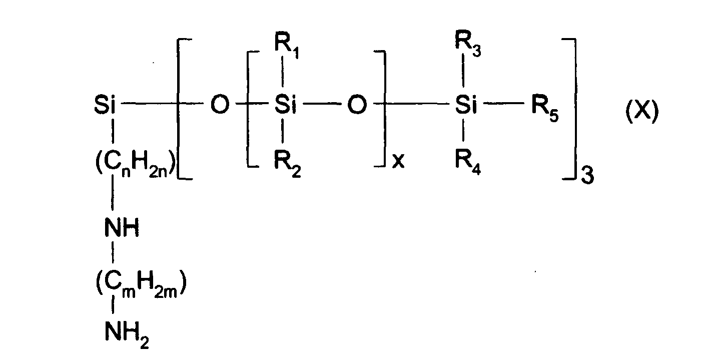 dans laquelle :
- R1, R2, R3 et R4, identiques ou différents, désignent un radical alkyle en C1-C4 ou un groupement phényle,
- R5 désigne un radical alkyle en C1-C4 ou un groupement hydroxyle,
- n est un entier variant de 1 à 5,
- m est un entier variant de 1 à 5,
et dans laquelle x est choisi de manière telle que l'indice d'amine soit compris entre 0,01 et 1 meq/g.

8. Composition selon la revendication 7, **caractérisée en ce que** la silicone aminée de formule (IX) est la silicone dénommée « triméthylsilylamodiméthicone », répondant à la formule : 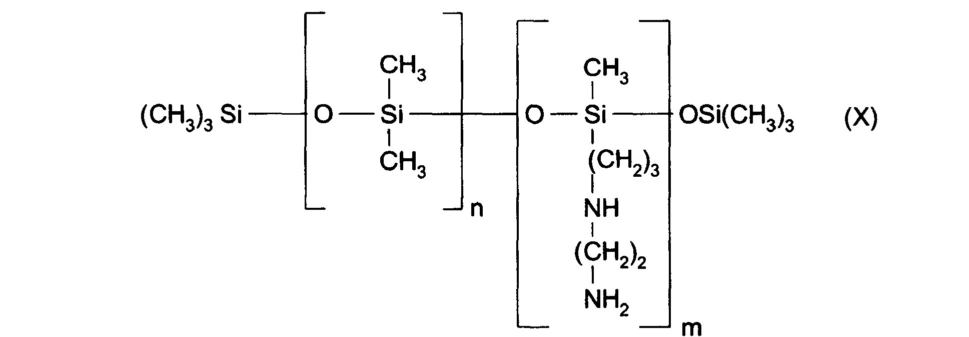 dans laquelle n et m ont les mêmes significations que dans la formule (IX).

9. Composition selon la revendication 1, **caractérisée en ce que** la silicone aminée est choisie parmi les amodiméthicones et les triméthylsilylamodiméthicones, les silicones à groupements ammonium quaternaire.

10. Composition selon l'une quelconque des revendications précédentes, **caractérisée en ce que** la silicone aminée est présente dans la composition à une teneur comprise entre 0,01 et 6%, de préférence entre 0,1 et 3%, et encore de préférence entre 0,4 et 2% en poids du poids total de la composition.

11. Composition selon l'une quelconque des revendications précédentes, **caractérisée en ce que** l'alcool gras est présent dans la composition à une teneur allant de 0,1 à 15%, de préférence de 0,5 à 10%, encore de préférence de 2 à 8 % en poids du poids total de la composition.

12. Composition selon l'une quelconque des revendications précédentes, **caractérisée en ce que** le diol est choisi parmi l'hexanediol-1,6, le dipropylèneglycol et leurs mélanges.

13. Composition selon l'une quelconque des revendications précédentes, **caractérisée en ce que** le diol est présent dans la composition à une teneur allant de 0,5 à 3% en poids du poids total de la composition.

14. Composition selon l'une quelconque des revendications 1 à 13, **caractérisée en ce que** le rapport des concentrations en poids alcool gras/tensioactif cationique va de préférence de 1 à 10 et plus particulièrement de 1 à 5.

15. Composition selon l'une quelconque des revendications 1 à 14, **caractérisée en ce que** le rapport des concentrations en poids tensioactif cationique/silicone va de préférence de 0,85 à 10 et plus particulièrement de 0,85 à 5.

16. Composition selon l'une quelconque des revendications précédentes, **caractérisée en ce que** le milieu cosmétiquement acceptable est constitué par de l'eau ou par un ou plusieurs solvants cosmétiquement acceptables ou par des mélanges eau-solvant(s).

17. Composition selon la revendication 16, **caractérisée en ce que** le solvant cosmétiquement acceptable est choisi parmi l'éthanol et l'isopropanol.

18. Composition selon l'une quelconque des revendications précédentes, **caractérisée en ce qu'**elle contient en outre au moins un additif choisi parmi les parfums, les filtres UV, les tensioactifs non ioniques, anioniques, amphotères ou zwitterioniques, les conservateurs, les protéines, les vitamines, les polymères non ioniques, anioniques, cationiques, amphotères ou zwitterioniques, les agents antipelliculaires, les agents anti-chute des cheveux, les colorants, les pigments, les réducteurs, les cires végétales, les céramides, les provitamines, les polymères non ioniques, anioniques, cationiques, amphotères ou zwitterioniques, les huiles minérales, végétales ou synthétiques, les silicones non aminées, les cires végétales, les céramides, les polyols autres que ceux de l'invention et tout autre additif classiquement utilisé dans les compositions cosmétiques.

19. Utilisation d'une composition selon l'une quelconque des revendications 1 à 18 pour le traitement cosmétique des cheveux et du cuir chevelu.

20. Procédé de traitement cosmétique des cheveux, **caractérisé en ce qu'**on applique sur les cheveux une composition cosmétique selon l'une des revendications 1 à 18.

21. Procédé selon la revendication 20, **caractérisé en ce que** l'application s'effectue après un shampooing.

## Claims

1. Non-colouring cosmetic composition, in particular a hair composition, **characterized in that** it comprises, in a cosmetically acceptable medium, at least one cationic surfactant chosen from quaternary ammonium salts, at least one aminated silicone, at least one fatty alcohol and at least one diol having 6 carbon atoms, the ratio of the fatty alcohol/cationic surfactant concentrations by weight being greater than or equal to 1, the ratio of the cationic surfactant/silicone concentrations by weight being greater than or equal to 0.85 and the ratio of the fatty alcohol/silicone concentrations by weight ranging from 2 to 5,
the diol being present in the composition at a content ranging from 0.25 to 5% by weight of the total weight of the composition,
the fatty alcohol being chosen from cetyl alcohol, stearyl alcohol, oleyl alcohol and their mixtures,
the composition comprising less than 0.5% by weight of oxidation dye, with respect to the total weight of the composition.

2. Composition according to Claim 1, **characterized in that** the quaternary ammonium salts are chosen from:
- those which exhibit the following general formula (I): 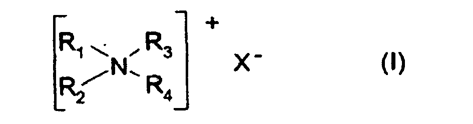 in which the symbols R₁ to R₄, which can be identical or different, represent a linear or branched aliphatic radical comprising from 1 to 30 carbon atoms or an aromatic radical, such as aryl or alkylaryl; X⁻ is an anion chosen from the group of the halides, phosphates, acetates, lactates, (C₂-C₆)alkyl sulphates, or alkyl- or alkylarylsulphonates;
- imidazoline quaternary ammonium salts of following formula (II): 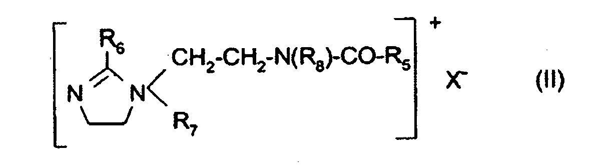 in which R₅ represents an alkenyl or alkyl radical comprising from 8 to 30 carbon atoms, R₆ represents a hydrogen atom, a C₁-C₄ alkyl radical or an alkenyl or alkyl radical comprising from 8 to 30 carbon atoms, R₇ represents a C₁-C₄ alkyl radical, R₈ represents a hydrogen atom or a C₁-C₄ alkyl radical and X⁻ is an anion chosen from the group of the halides, phosphates, acetates, lactates, alkyl sulphates, or alkyl- or alkylarylsulphonates;
- di(quaternary ammonium) salts of formula (III): 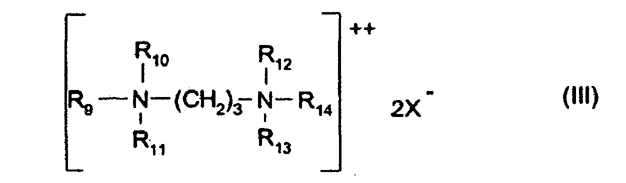 in which R₉ denotes an aliphatic radical comprising from 16 to 30 carbon atoms, R₁₀, R₁₁, R₁₂, R₁₃ and R₁₄, which are identical or different, are chosen from hydrogen or an alkyl radical comprising from 1 to 4 carbon atoms and X⁻ is an anion chosen from the group of the halides, acetates, phosphates, nitrates, ethyl sulphates and methyl sulphates;
- quaternary ammonium salts comprising at least one ester functional group of following formula (IV): 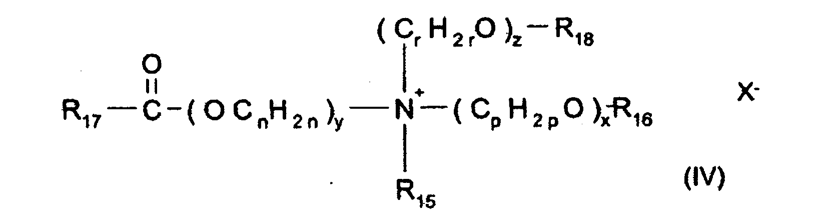 in which:
R₁₅ is chosen from C₁-C₆ alkyl radicals and C₁-C₆ hydroxyalkyl or dihydroxyalkyl radicals;
R₁₆ is chosen from:
- the 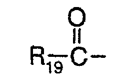 radical,
- saturated or unsaturated, linear or branched, C₁-C₂₂ hydrocarbon radicals R₂₀,
- the hydrogen atom,
R₁₈ is chosen from:
- the 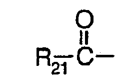 radical,
- saturated or unsaturated, linear or branched, C₁-C₆ hydrocarbon radicals R₂₂,
- the hydrogen atom,
R₁₇, R₁₉ and R₂₁, which are identical or different, are chosen from saturated or unsaturated, linear or branched, C₇-C₂₁ hydrocarbon radicals;
r, n and p, which are identical or different, are integers having values from 2 to 6;
y is an integer having a value from 1 to 10;
x and z, which are identical or different, are integers having values from 0 to 10;
X⁻ is an organic or inorganic, simple or complex anion;
with the proviso that the sum x + y + z has a value from 1 to 15, that when x has a value of 0, then R₁₆ denotes R₂₀, and that when z has a value of 0, then R₁₈ denotes R₂₂.

3. Composition according to Claim 2, **characterized in that** the compound of formula (IV) is chosen from diacyloxyethyldimethylammonium, diacyloxyethyl(hydroxyethyl)methylammonium, monoacyloxyethyldi(hydroxyethyl)-methylammonium, triacyloxyethylmethylammonium or monoacyloxyethyl(hydroxyethyl)dimethylammonium salts and their mixtures.

4. Composition according to Claim 2, **characterized in that** the surfactant of formula (I) is chosen from dipalmitoylethylhydroxyethylmethylammonium salts, behenyltrimethylammonium, distearyldimethylammonium, cetyltrimethylammonium or benzyldimethylstearylammonium salts, palmitylamidopropyltrimethylammonium salts or stearamidopropyldimethyl(myristyl acetate)ammonium salts.

5. Composition according to any one of the preceding claims, **characterized in that** the cationic surfactant is chosen from behenyltrimethylammonium chloride, cetyltrimethylammonium chloride, quaternium-83, behenylamidopropyl(2,3-dihydroxypropyl)dimethylammonium chloride and palmitylamidopropyltrimethylammonium chloride.

6. Composition according to any one of the preceding claims, **characterized in that** the cationic surfactant is present in the composition at a content of between 0.01 and 10%, preferably between 0.1 and 5% and more preferably between 0.2 and 4%, by weight of the total weight of the composition.

7. Composition according to any one of the preceding claims, **characterized in that** the aminated silicone is chosen from:
a) the polysiloxanes named "amodimethicone" in the CTFA dictionary and corresponding to the formula: 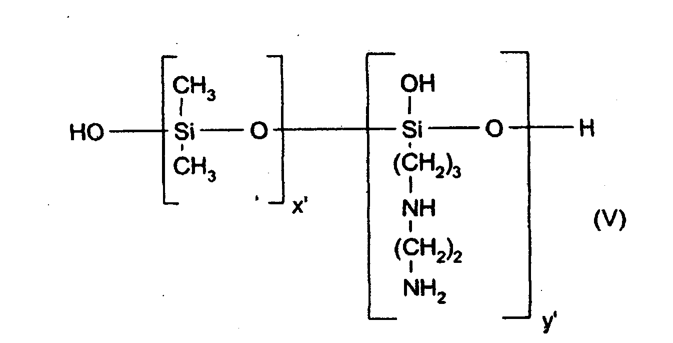 in which x' and y' are integers such that the weight-average molecular weight is between 5000 and 500 000;
b) the aminated silicones corresponding to the formula:
R'ₐG₃₋ₐ-Si(OSiG₂)ₙ-(OSiG_{b}R'_{2-b})ₘ-O-SiG₃₋ₐ-R'ₐ (VI)
in which:
G is a hydrogen atom or a phenyl, OH or C₁-C₈ alkyl group,
a denotes the number 0 or an integer from 1 to 3,
b denotes 0 or 1,
m and n are numbers such that the sum (n + m) can vary in particular from 1 to 2000 and especially from 50 to 150, it being possible for n to denote a number from 0 to 1999 and in particular from 49 to 149 and it being possible for m to denote a number from 1 to 2000 and in particular from 1 to 10,
R' is a monovalent radical of formula -C_{q}H_{2q}L in which q is a number from 2 to 8 and L is an optionally quaternized amino group chosen from the groups:
-NR"-Q-N'(R")₂
-N(R")₂
-N⁺(R")₃ A⁻
-NH⁺(R")₂ A⁻
-NH₂⁺(R") A⁻
-N(R")-Q-N+R"H₂ A⁻
-NR"-Q-N+(R")₂H A⁻
-NR"-Q-N⁺(R")₃ A⁻
in which R" can denote hydrogen, phenyl, benzyl or a saturated monovalent hydrocarbon radical; Q denotes a linear or branched group of formula CᵣH₂ᵣ, r being an integer ranging from 2 to 6, preferably from 2 to 4, and A⁻ represents a halide ion,
c) the aminated silicones corresponding to the formula: 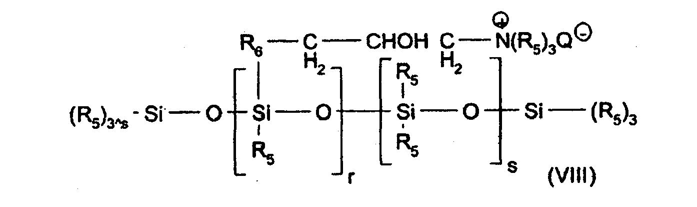 in which:
R₅ represents a monovalent hydrocarbon radical having from 1 to 18 carbon atoms and in particular a C₁-C₁₈ alkyl or C₂-C₁₈ alkenyl radical;
R₆ represents a divalent hydrocarbon radical, in particular a C₁-C₁₈ alkylene radical, or a divalent C₁-C₁₈ alkyleneoxy radical;
Q⁻ is an anion, such as a halide ion or an organic acid salt;
r represents a mean statistical value from 2 to 20 and in particular from 2 to 8;
s represents a mean statistical value from 20 to 200 and in particular from 20 to 50,
d) the quaternary ammonium silicones of formula: 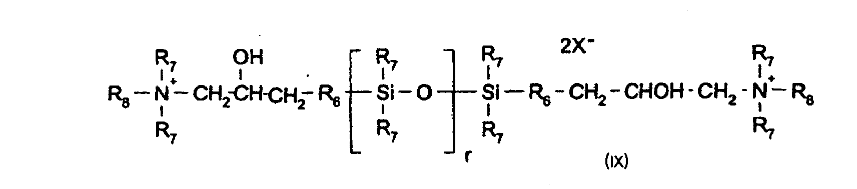 in which:
R₇, which are identical or different, represent a monovalent hydrocarbon radical having from 1 to 18 carbon atoms and in particular a C₁-C₁₈ alkyl radical, a C₂-C₁₈ alkenyl radical or a ring comprising 5 or 6 carbon atoms;
R₆ represents a divalent hydrocarbon radical, in particular a C₁-C₁₈ alkylene radical, or a divalent C₁-C₁₈ alkyleneoxy radical;
R₈, which are identical or different, represent a hydrogen atom, a monovalent hydrocarbon radical having from 1 to 18 carbon atoms, in particular a C₁-C₁₈ alkyl radical or a C₂-C₁₈ alkenyl radical, or an -R₆-NHCOR₇ radical;
X⁻ is an anion, such as a halide ion or an organic acid salt;
r represents a mean statistical value from 2 to 200 and in particular from 5 to 100;
e) the aminated silicones of formula (X): 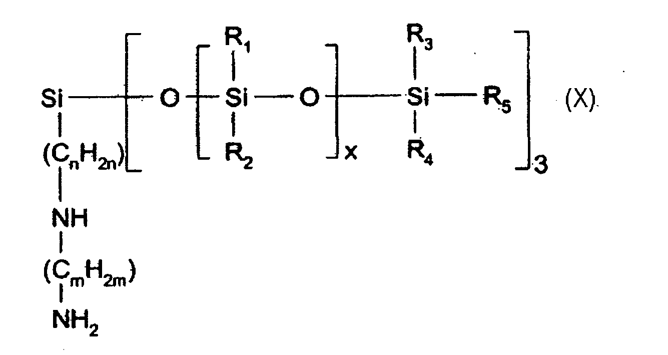 in which:
- R₁, R₂, R₃ and R₄, which are identical or different, denote a C₁-C₄ alkyl radical or a phenyl group,
- R₅ denotes a C₁-C₄ alkyl radical or a hydroxyl group,
- n is an integer varying from 1 to 5,
- m is an integer varying from 1 to 5,
and in which x is chosen so that the amine number is between 0.01 and 1 meq/g.

8. Composition according to Claim 7, **characterized in that** the aminated silicone of formula (VI) is the silicone named "trimethylsilylamodimethicone", corresponding to the formula: 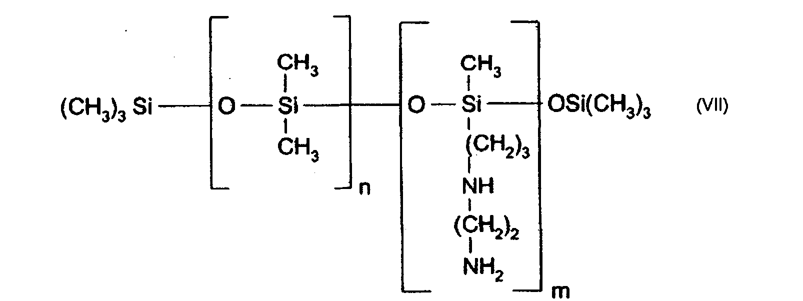 in which n and m have the same meanings as in the formula (VI).

9. Composition according to Claim 1, **characterized in that** the aminated silicone is chosen from amodimethicones and trimethylsilylamodimethicones, or silicones comprising quaternary ammonium groups.

10. Composition according to any one of the preceding claims, **characterized in that** the aminated silicone is present in the composition at a content of between 0.01 and 6%, preferably between 0.1 and 3% and more preferably between 0.4 and 2%, by weight of the total weight of the composition.

11. Composition according to any one of the preceding claims, **characterized in that** the fatty alcohol is present in the composition at a content ranging from 0.1 to 15%, preferably from 0.5 to 10%, more preferably from 2 to 8%, by weight of the total weight of the composition.

12. Composition according to any one of the preceding claims, **characterized in that** the diol is chosen from 1,6-hexanediol, dipropylene glycol and their mixtures.

13. Composition according to any one of the preceding claims, **characterized in that** the diol is present in the composition at a content ranging from 0.5 to 3%, by weight of the total weight of the composition.

14. Composition according to any one of Claims 1 to 13, **characterized in that** the ratio of the fatty alcohol/cationic surfactant concentrations by weight preferably ranges from 1 to 10 and more particularly from 1 to 5.

15. Composition according to any one of Claims 1 to 14, **characterized in that** the ratio of the cationic surfactant/silicone concentrations by weight preferably ranges from 0.85 to 10 and more particularly from 0.85 to 5.

16. Composition according to any.one of the preceding claims, **characterized in that** the cosmetically acceptable medium is composed of water or of one or more cosmetically acceptable solvents or of water-solvent(s) mixtures.

17. Composition according to Claim 16, **characterized in that** the cosmetically acceptable solvent is chosen from ethanol and isopropanol.

18. Composition according to any one of the preceding claims, **characterized in that** it additionally comprises at least one additive chosen from fragrances, UV screening agents, nonionic, anionic, amphoteric or zwitterionic surfactants, preservatives, proteins, vitamins, nonionic, anionic, cationic, amphoteric or zwitterionic polymers, antidandruff agents, agents for combating hair loss, dyes, pigments, reducing agents, vegetable waxes, ceramides, provitamins, mineral, vegetable or synthetic oils, non-aminated silicones, polyols, other than those of the invention, and any other additive conventionally used in cosmetic compositions.

19. Use of a composition according to any one of Claims 1 to 18 in the cosmetic treatment of the hair and scalp.

20. Method for the cosmetic treatment of the hair, **characterized in that** a cosmetic composition according to one of Claims 1 to 18 is applied to the hair.

21. Method according to Claim 20, **characterized in that** the application is carried out after shampooing.

## Patentansprüche

1. Nichtfärbende kosmetische Zusammensetzung, insbesondere für die Haarbehandlung, **dadurch gekennzeichnet, dass** sie in einem kosmetisch akzeptablen Medium mindestens einen kationischen grenzflächenaktiven Stoff, der unter den quartären Ammoniumsalzen ausgewählt ist, mindestens ein aminiertes Silicon, mindestens einen Fettalkohol und mindestens ein Diol mit 6 Kohlenstoffatomen enthält, wobei das auf das Gewicht bezogene Verhältnis der Konzentration Fettalkohol/kationischer grenzflächenaktiver Stoff größer oder gleich 1 ist, das auf das Gewicht bezogene Verhältnis der Konzentration kationischer grenzflächenaktiver Stoff/Silicon größer oder gleich 0,85 ist und das auf das Gewicht bezogene Verhältnis der Konzentrationen Fettalkohol/Silicon im Bereich von 2 bis 5 liegt,
wobei das Diol in der Zusammensetzung in einem Mengenanteil von 0,25 bis 5 Gew.-% des Gesamtgewichts der Zusammensetzung enthalten ist,
wobei der Fettalkohol unter Cetylalkohol, Stearylalkohol, Oleylalkohol und deren Gemischen ausgewählt ist,
wobei die Zusammensetzung weniger als 0,5 Gew.-% Oxidationsfarbstoff, bezogen auf das Gesamtgewicht der Zusammensetzung, enthält.

2. Zusammensetzung nach Anspruch 1, **dadurch gekennzeichnet, dass** die quartären Ammoniumsalze ausgewählt sind unter:
- quartären Ammoniumsalzen, die die folgende allgemeine Formel (I) aufweisen: 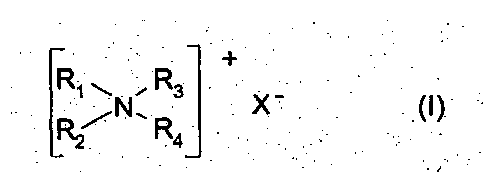 wobei in der Formel die Symbole R₁ bis R₄, die identisch oder voneinander verschieden sein können, eine lineare oder verzweigte, aliphatische Gruppe mit 1 bis 30 Kohlenstoffatomen oder eine aromatische Gruppe, wie Aryl oder Alkylaryl, bedeuten; X- ein Anion ist, das unter den Halogeniden, Phosphaten, Acetaten, Lactaten, Alkyl(C₂₋₆)sulfaten, Alkyl- oder Alkylarylsulfonaten ausgewählt ist;
- quartären Ammoniumsalzen von Imidazolin der folgenden Formel (II): 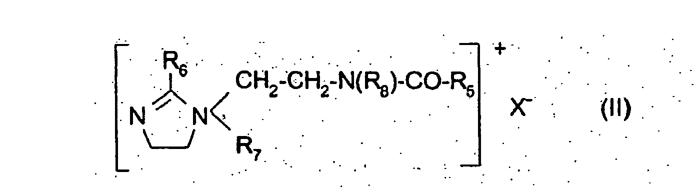 worin R₅ eine Alkenyl- oder Alkylgruppe mit 8 bis 30 Kohlenstoffatomen bedeutet, R₆ ein Wasserstoffatom, eine C₁₋₄-Alkylgruppe oder eine Alkenyl- oder Alkylgruppe mit 8 bis 30 Kohlenstoffatomen bedeutet, R₇ eine C₁₋₄-Alkylgruppe ist, A₈ ein Wasserstoffatom, eine C₁₋₄-Alkylgruppe bedeutet, X ein Anion ist, das unter den Halogeniden, Phosphaten, Acetaten, Lactaten, Alkylsulfaten, Alkyl- oder Alkylarylsulfonaten ausgewählt ist;
- quartären Diammoniumsalzen der Formel (III): 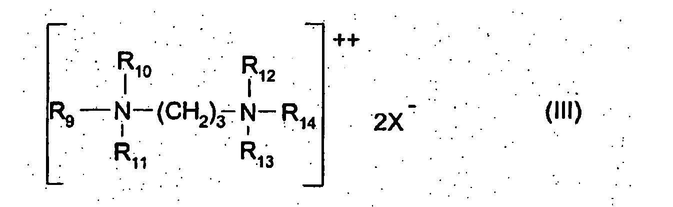 bei der Rg eine aliphatische Gruppe mit 16 bis 30 Kohlenstoffatomen bedeutet, R₁₀, R₁₁, R₁₂, R₁₃ und R₁₄, die gleich oder verschieden sind, unter Wasserstoff oder einer Alkylgruppe mit 1 bis 4 Kohlenstoffatomen ausgewählt sind, und X- ein Anion ist, das unter den Halogeniden, Acetaten, Phosphaten, Nitraten, Ethylsulfaten und Methylsulfaten ausgewählt ist;
- quartären Ammoniumsalzen, die mindestens eine Esterfunktion enthalten, der folgenden Formel (IV): 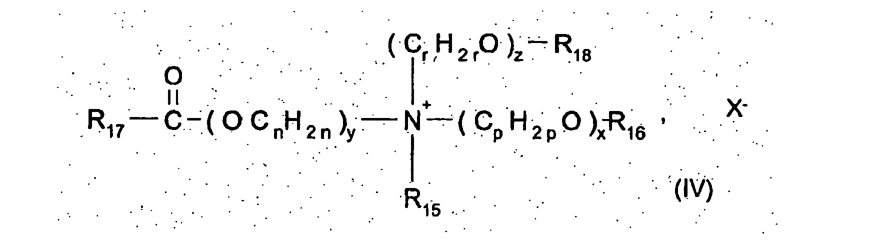 in der Formel:
R₁₅ ist unter den C₁₋₆-Alkylgruppen und den Hydroxyalkyl- oder Dihydroxyalkylgruppen mit 1 bis 6 Kohlenstoffatomen ausgewählt;
R₁₆ ist ausgewählt unter:
- der Gruppe 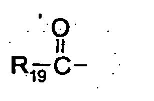
- den linearen oder verzweigten, gesättigten oder ungesättigten Gruppen R₂₀ auf Kohlenwasserstoffbasis mit 1 bis 22 Kohlenstoffatomen,
- dem Wasserstoffatom,
R₁₈ ist ausgewählt unter:
- der Gruppe 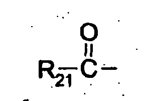
- den linearen oder verzweigten, gesättigten oder ungesättigten Gruppen R₂₂ auf Kohlenwasserstoffbasis mit 1 bis 6 Kohlenstoffatomen
- dem Wasserstoffatom,
R₁₇, R₁₉ und R₂₁, die gleich oder verschieden sind, sind unter den linearen oder verzweigten, gesättigten oder ungesättigten
Gruppen auf Kohlenwasserstoffbasis mit 7 bis 21 Kohlenstoffatomen ausgewählt;
r, n und p, die gleich oder verschieden sind, sind ganze Zahlen im Bereich von 2 bis 6;
y ist eine ganze Zahl im Bereich von 1 bis 10;
x und z, die gleich oder verschieden sind, bedeuten 0 oder ganze Zahlen von 1 bis 10;
X- ist ein einfaches oder komplexes, organisches oder anorganisches Anion;
mit der Maßgabe, dass sie Summe x + y + z im Bereich von 1 bis 15 liegt, R₁₆ R₂₀ bedeutet, wenn x 0 ist, und R₁₈ R₂₂ bedeutet, wenn z 0 ist.

3. Zusammensetzung nach Anspruch 2, **dadurch gekennzeichnet, dass** die Verbindung der Formel (IV) unter den Diacyloxyethyldimethylammoniumsalzen, Diacyloxyethyl-hydroxyethyl-methylammoniumsalzen, Monoacyloxyethyl-dihydroxyethyl-methylammoniumsalzen, Triacyloxyethyl-methylammoniumsalzen, Monoacyloxyethyl-hydroxyethyl-dimethylammoniumsalzen und deren Gemischen ausgewählt ist.

4. Zusammensetzung nach Anspruch 2, **dadurch gekennzeichnet, dass** der grenzflächenaktive Stoff der Formel (I) unter den Dipalmitoylethylhydroxyethylmethylammoniumsalzen, Behenyltrimethylammoniumsalzen, Distearyldimethylammoniumsalzen, Cetyltrimethylammoniumsalzen, Benzyldimethylstearylammoniumsalzen, Palmitylamidopropyltrimethylammoniumsalzen, Stearamidopropyldimethyl(myristylacetat)ammoniumsalzen ausgewählt ist.

5. Zusammensetzung nach einem der vorhergehenden Ansprüche, **dadurch gekennzeichnet, dass** der kationische grenzflächenaktive Stoff unter Behenyltrimethylammoniumchlorid, Cetyltrimethylammoniumchlorid, Quaternium-83, Behenylamidopropyl-2,3-dihydroxypropyldimethylammoniumchlorid und Palmitylamidopropyltrimethylammoniumchlorid ausgewählt ist.

6. Zusammensetzung nach einem der vorhergehenden Ansprüche, **dadurch gekennzeichnet, dass** der kationische grenzflächenaktive Stoff in der Zusammensetzung in einem Mengenanteil von 0,01 bis 10 %, vorzugsweise 0,1 bis 5 % und noch bevorzugter 0,2 bis 4 Gew.-% des Gesamtgewichts der Zusammensetzung enthalten ist.

7. Zusammensetzung nach einem der vorhergehenden Ansprüche, **dadurch gekennzeichnet, dass** das aminierte Silicon ausgewählt ist unter:
a) Polysiloxanen, die nach CTFA-Lexikon als "Amodimethicone" bezeichnet werden und der folgenden Formel entsprechen: 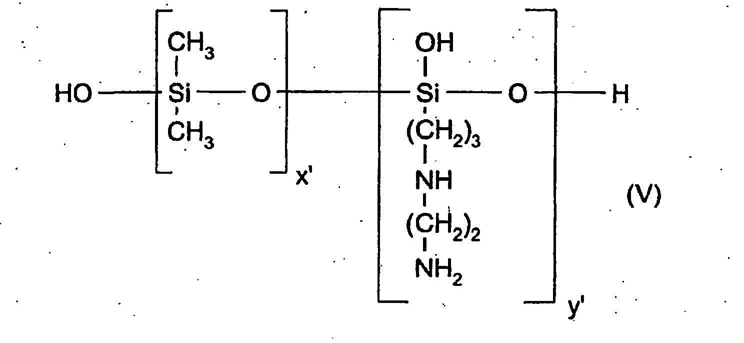 worin x' und y' ganze Zahlen sind, die so gewählt sind, dass die gewichtsmittlere Molmasse im Bereich von 5 000 bis 500 000 liegt;
b) aminierten Siliconen der Formel:
R'aG3-a-Si(OSiG2)n-(OSiGbR'2-b)m-O-SiG3-a-R'a (VI)
worin bedeuten:
G ein Wasserstoffatom, eine Phenylgruppe, OH oder C₁₋₈-Alkyl,
a 0 oder eine ganze Zahl von 1 bis 3,
b 0 oder 1,
m und n Zahlen, die so sind, dass die Summe (n + m) insbesondere im Bereich von 1 bis 2 000 und besonders 50 bis 150 liegen kann, wobei n eine Zahl von 0 bis 1 999 und insbesondere 49 bis 149 bedeuten kann und m eine Zahl von 1 bis 2 000 und insbesondere 1 bis 10 sein kann;
R' eine einwertige Gruppe der Formel -CqH₂qL, worin q eine ganze Zahl von 2 bis 8 ist und L eine gegebenenfalls quatemisierte aminierte Gruppe ist, die unter den folgenden Gruppen ausgewählt ist:
-NR"-Q-N'(R")2
-N(R")2
-N⊕(R")3 A-
-NH⊕(R")2 A-
-NH2⊕(R") A-
-N(R")-Q-N⊕R"H2 A-
-NR"-Q-N⊕(R")2H A-
-NR"-Q-N⊕(R")3 A-
worin R" Wasserstoff, Phenyl, Benzyl oder eine gesättigte einwertige Kohlenwasserstoffgruppe bedeuten kann; Q eine geradkettige oder verzweigte Gruppe der Formel CᵣH₂ᵣ ist, wobei r eine ganze Zahl von 2 bis 6 und vorzugsweise 2 bis 4 bedeutet; und A- ein Halogenid bedeutet,
c) aminierten Siliconen der folgenden Formel: 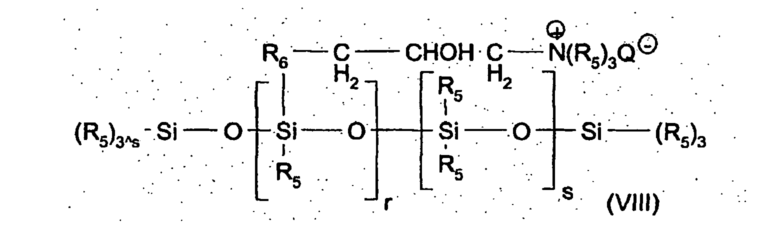 wobei in der Formel:
R₅ bedeutet eine einwertige Kohlenwasserstoffgruppe mit 1 bis 18 Kohlenstoffatomen und insbesondere C₁₋₁₈-Alkyl oder C₂₋₁₈-Alkenyl;
R₆ bedeutet eine zweiwertige Kohlenwasserstoffgruppe und insbesondere eine Alkylengruppe mit 1 bis 18 Kohlenstoffatomen oder eine zweiwertige Alkylenoxygruppe mit 1 bis 18 Kohlenstoffatomen;
Q- ist ein Anion, beispielsweise ein Halogenid oder das Salz einer organischen Säure;
r bedeutet einen statistischen Mittelwert von 2 bis 20 und insbesondere 2 bis 8;
s bedeutet einen statistischen Mittelwert von 20 bis 200 und insbesondere 20 bis 50;
d) quartären Ammoniumsiliconen der folgenden Formel: 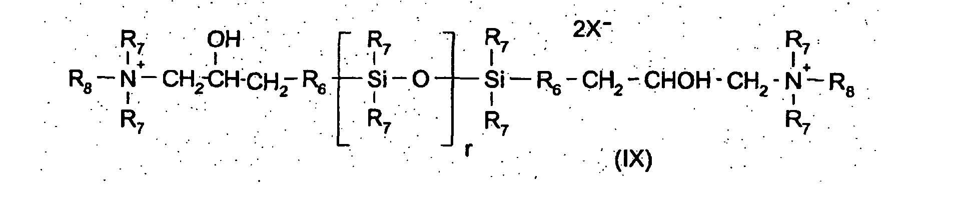 wobei in der Formel:
R₇, die gleich oder verschieden sind, bedeuten eine einwertige
Kohlenwasserstoffgruppe mit 1 bis 18 Kohlenstoffatomen, insbesondere C₁₋₁₈-Alkyl, C₂₋₁₈-Alkenyl oder einen Ring mit 5 oder 6 Kohlenstoffatomen;
R₆ bedeutet eine zweiwertige Kohlenwasserstoffgruppe, insbesondere C₁₋₁₈-Alkylen oder eine zweiwertige Alkylenoxygruppe mit 1 bis 18 Kohlenstoffatomen;
die Gruppen R₈, die gleich oder verschieden sind, bedeuten
ein Wasserstoffatom, eine einwertige Kohlenwasserstoffgruppe mit 1 bis 18 Kohlenstoffatomen, insbesondere C₁₋₁₈-Alkyl, C₂₋₁₈-Alkenyl, oder R₆-NHCOR₇;
X- ist ein Anion, beispielsweise ein Halogenid oder ein organisches Säuresalz;
r bedeutet einen statistischen Mittelwert von 2 bis 200 und insbesondere 5 bis 100;
e) aminierten Siliconen der Formel (X): 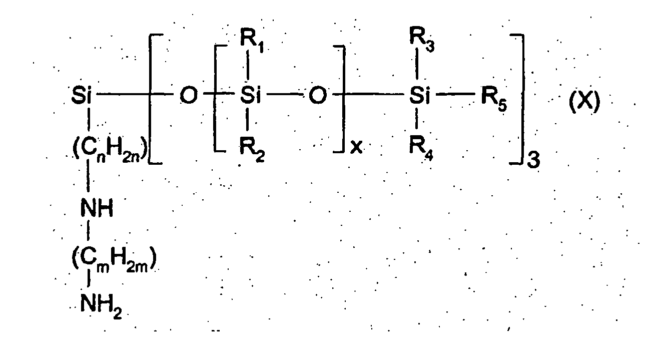 wobei in der Formel:
- R₁, R₂, R₃ und R₄, die gleich oder verschieden sind, bedeuten eine C₁₋₄-Alkylgruppe oder eine Phenylgruppe,
- R₅ bedeutet eine C₁₋₄-Alkylgruppe oder eine Hydroxygruppe,
- n ist eine ganze Zahl von 1 bis 5,
- m ist eine ganze Zahl von 1 bis 5,
wobei x so gewählt ist, dass der Amin-Index im Bereich von 0,01 bis 1 meq/g liegt.

8. Zusammensetzung nach Anspruch 7, **dadurch gekennzeichnet, dass** das aminierte Silicon der Formel (IX) das als «Trimethylsilylamodimethicon» bezeichnete Silicon ist, das der folgenden Formel entspricht: 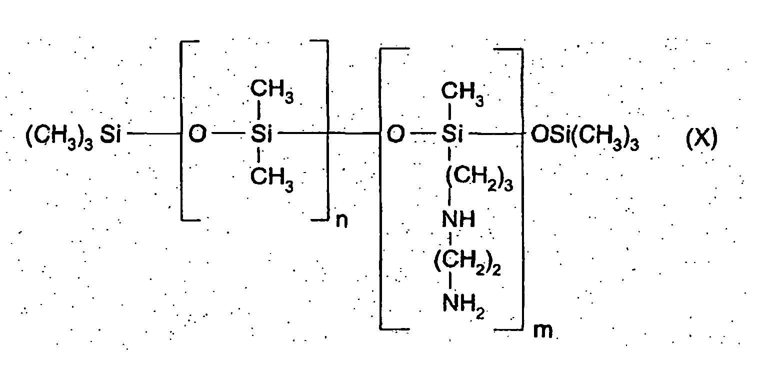 worin n und m die oben für die Formel (IX) angegebenen Bedeutungen aufweisen.

9. Zusammensetzung nach Anspruch 1, **dadurch gekennzeichnet, dass** das aminierte Silicon unter den Amodimethiconen und Trimethylsilylamodimethiconen, Siliconen mit quartären Ammoniumgruppen ausgewählt ist.

10. Zusammensetzung nach einem der vorhergehenden Ansprüche, **dadurch gekennzeichnet, dass** das aminierte Silicon in der Zusammensetzung in einer Menge von 0,01 bis 6 %, vorzugsweise 0,1 bis 3 % und noch bevorzugter 0,4 bis 2 Gew.-% des Gesamtgewichts der Zusammensetzung enthalten ist.

11. Zusammensetzung nach einem der vorhergehenden Ansprüche, **dadurch gekennzeichnet, dass** der Fettalkohol in der Zusammensetzung in einem Mengenanteil von 0,1 bis 15 %, vorzugsweise 0,5 bis 10 % und noch bevorzugter 2 bis 8 Gew.-% des Gesamtgewichts der Zusammensetzung enthalten ist.

12. Zusammensetzung nach einem der vorhergehenden Ansprüche, **dadurch gekennzeichnet, dass** das Diol unter 1,6-Hexandiol, Dipropylenglycol und deren Gemischen ausgewählt ist.

13. Zusammensetzung nach einem der vorhergehenden Ansprüche, **dadurch gekennzeichnet, dass** das Diol in der Zusammensetzung in einem Mengenanteil von 0,5 bis 3 Gew.-% des Gesamtgewichts der Zusammensetzung enthalten ist.

14. Zusammensetzung nach einem der Ansprüche 1 bis 13, **dadurch gekennzeichnet, dass** das auf das Gewicht bezogene Verhältnis der Konzentration Fettalkohol/kationischer grenzflächenaktiver Stoff vorzugsweise im Bereich von 1 bis 10 und insbesondere 1 bis 5 liegt.

15. Zusammensetzung nach einem der Ansprüche 1 bis 14, **dadurch gekennzeichnet, dass** das auf das Gewicht bezogene Verhältnis der Konzentration kationischer grenzflächenaktiver Stoff/Silicon vorzugsweise im Bereich von 0,85 bis 10 und insbesondere 0,85 bis 5 liegt.

16. Zusammensetzung nach einem der vorhergehenden Ansprüche, **dadurch gekennzeichnet, dass** das kosmetisch akzeptable Medium aus Wasser oder aus einem oder mehreren kosmetisch akzeptablen Lösungsmitteln oder aus Gemischen Wasser-Lösungsmittel(n) besteht.

17. Zusammensetzung nach Anspruch 16, **dadurch gekennzeichnet, dass** das kosmetisch akzeptable Lösungsmittel unter Ethanol und Isopropanol ausgewählt ist.

18. Zusammensetzung nach einem der vorhergehenden Ansprüche, **dadurch gekennzeichnet, dass** sie ferner mindestens einen Zusatzstoff enthält, der unter den Parfums, UV-Filtern, nichtionischen, anionischen, amphoteren oder zwitterionischen grenzflächenaktiven Stoffen, Konservierungsmitteln, Proteinen, Vitaminen, nichtionischen, anionischen, kationischen, amphoteren oder zwitterionischen Polymeren, Antischuppenmitteln, Wirkstoffen gegen Haarausfall, Farbstoffen, Pigmenten, Reduktionsmitteln, pflanzlichen Wachsen, Ceramiden, Provitaminen, nichtionischen, anionischen, kationischen, amphoteren oder zwitterionischen Polymeren, Mineralölen, pflanzlichen Ölen oder synthetischen Ölen, nicht aminierten Siliconen, pflanzlichen Wachsen, Ceramiden, Polyolen, die von den erfindungsgemäßen Polyolen verschieden sind, und allen anderen, herkömmlich in kosmetischen Zusammensetzungen verwendeten Zusatzstoffen ausgewählt ist.

19. Verwendung einer Zusammensetzung nach einem der Ansprüche 1 bis 18 für die kosmetische Behandlung der Haare und der Kopfhaut.

20. Verfahren zur kosmetischen Behandlung der Haare, **dadurch gekennzeichnet, dass** auf die Haare eine kosmetische Zusammensetzung nach einem der Ansprüche 1 bis 18 aufgebracht wird.

21. Verfahren nach Anspruch 20, **dadurch gekennzeichnet, dass** die Anwendung nach einer Haarwäsche erfolgt.
